# DEMANDE DE BREVET EUROPEEN

(11) **EP 0 559 572 A1**
(43) Date de publication de la demande: **08.09.1993**
(21) Numéro de dépôt: 93400569.5
(22) Date de dépôt: 05.03.1993
(51) Int. Cl.: G01N 33/569

(54) **Procédé de contrôle préventif de l'immunité chez le sujet humain par l'étude de la population lymphocytaire**

(30) Priorité: 06.03.1992 FR 9202713
(71) Demandeur: Donzeau, Jean-Claude, F-31400 Toulouse (FR)
(72) Inventeur: Donzeau, Jean-Claude, F-31400 Toulouse (FR)
(74) Mandataire: Kedinger, Jean-Paul

(57) **Abrégé**

Procédé de contrôle préventif de l'immunité chez le sujet humain pour détecter une éventuelle déficience du système immunitaire, qui comprend les étapes consistant à :
(a) établir la courbe de l'évolution du rapport nombre de lymphocytes T4/nombre de lymphocytes T8 en fonction de l'âge sur des échantillons sanguins prélevés sur des sujets humains sains,
(b) déterminer, dans les mêmes conditions que (a), le rapport nombre de lymphocytes T4/nombre de lymphocytes T8 sur un échantillon sanguin prélevé sur un sujet humain en examen, et
(c) comparer le rapport nombre de lymphocytes T4/nombre de lymphocytes T8 obtenu à l'étape (b) à la courbe obtenue à l'étape (a), un rapport nombre de lymphocytes T4/nombre de lymphocytes T8 non en accord avec cette courbe étant révélateur d'une déficience du système immunitaire du sujet en examen.

## Description

La présente invention a pour objet un procédé de contrôle préventif de l'immunité chez le sujet humain pour détecter une éventuelle déficience du système immunitaire.

L'évolution des techniques d'analyse a permis la détermination qualitative et quantitative d'entités les plus diverses. Tel est notamment le cas dans le domaine médical où l'on dispose aujourd'hui de méthodes et d'appareils d'analyse les plus sophistiqués permettant le contrôle, la surveillance et l'évolution d'innombrables paramètres biologiques et, partant, la détection d'une anomalie éventuelle de ces paramètres.

A ce jour toutefois, il n'a pas été effectué d'études systématiques du paramètre immunitaire que constitue la population lymphocytaire et ce, malgré le fait qu'il existe un certain nombre de techniques (immunoanalyse et cytométrie en flux par exemple) permettant d'appréhender ce type de population. Ainsi, bien que l'on dispose de moyens techniques d'exploration de la population lymphocy-taire, on ne sait pas aujourd'hui tirer partie de ces moyens pour établir une méthode d'appréciation de l'état immunitaire d'un individu.

Or, il est d'un intérêt tout à fait évident de pouvoir disposer d'un procédé rapide, sûr et de mise en oeuvre aisée, de mise en évidence des syndromes immunodéficients, avant qu'ils ne soient définitivement acquis ou encore des syndromes immunitaires avant qu'ils ne soient déficients.

Le but de la présente invention est donc la mise au point d'un tel procédé.

Dans cette optique, il a été effectué une étude systématique des populations lymphocytaires B, T et NK, sur des sujets sains et de tous âges, en faisant appel à la cytométrie en flux, technique bien connue et capable d'approcher les différentes classes de différenciation (CD) des lymphocytes B, T et NK. Les résultats obtenus dans le cadre de cette étude ont permis de mettre en évidence que le nombre de lymphocytes T porteurs du marqueur de surface CD4 (appelés lymphocytes T4), le nombre de lymphocytes T porteurs du marqueur de surface CD8 (appelés lymphocytes T8) et le rapport T4/T8 n'étaient pas constants mais subissaient des variations en fonction de l'âge des sujets testés. Ces résultats sont présentés ci-après et expriment les moyennes des valeurs obtenues chez environ 1200 sujets sur le sang total périphérique au cytomètre de flux EPICS PROFILE II (marque de fabrique de la société américaine COULTER INC.).

| Nouveaux nés (moyenne des valeurs obtenues sur 35 sujets) | | |
|---|---|---|
| Nombre de T4 par mm³ de sang total (± écart type) | Nombre de T8 par mm³ de sang total (± écart type) | T4/T8 (± écart type) |
| 3358 (± 1551) | 1313 (± 836) | 2,83 (± 0,76) |

| Sujets de 0-4 ans (moyenne des valeurs obtenues sur 87 sujets) | | | |
|---|---|---|---|
| Age moyen (ans) | Nombre de T4 par mm³ de sang total (± écart type) | Nombre de T8 par mm³ de sang total (± écart type) | T4/T8 (± écart type) |
| 2 | 2618 (± 961) | 1496 (± 724) | 1,92 (± 0,66) |

| Sujets de 20-40 ans (moyenne des valeurs obtenues sur 292 sujets) | | | |
|---|---|---|---|
| Age moyen (ans) | Nombre de T4 par mm³ de sang total (± écart type) | Nombre de T8 par mm³ de sang total (± écart type) | T4/T8 (± écart type) |
| 30 | 1554 (± 705) | 853 (± 317) | 1,80 (±0,27) |

| Sujets de 40-60 ans (moyenne des valeurs obtenues sur 418 sujets) | | | |
|---|---|---|---|
| Age moyen (ans) | Nombre de T4 par mm³ de sang total (± écart type) | Nombre de T8 par mm³ de sang total (± écart type) | T4/T8 (± écart type) |
| 52 | 1339 (± 432) | 581 (± 216) | 2,38 (± 0,30) |

| Sujets de 60-80 ans (moyenne des valeurs obtenues sur 197 sujets) | | | |
|---|---|---|---|
| Age moyen (ans) | Nombre de T4 par mm³ de sang total (± écart type) | Nombre de T8 par mm³ de sang total (± écart type) | T4/T8 (± écart type) |
| 67 | 1046 (± 326) | 432 (± 167) | 2,62 (± 0,99) |

| Sujets de plus de 80 ans (moyenne des valeurs obtenues sur 135 sujets) | | | |
|---|---|---|---|
| Age moyen (ans) | Nombre de T4 par mm³ de sang total (± écart type) | Nombre de T8 par mm³ de sang total (± écart type) | T4/T8 (± écart type) |
| 84 | 1297 (± 400) | 453 (± 148) | 2,93 (± 0,56) |

A partir des chiffres ci-dessus et du nombre de mesures efffectuées pour chaque catégorie de sujet, on estime que la limite de précision pour chacun des rapports moyens T4/T8 déterminés et figurant dans les tableaux ci-dessus est de ± 10 %.

A partir des résultats ci-dessus, il est possible d'établir deux courbes dites du vieillissement immunologique représentant respectivement le nombre de lymphocytes T4 et le nombre de lymphocytes T8 en fonction de l'âge et une courbe dite de l'immunité représentant le rapport T4/T8 en fonction le l'âge.

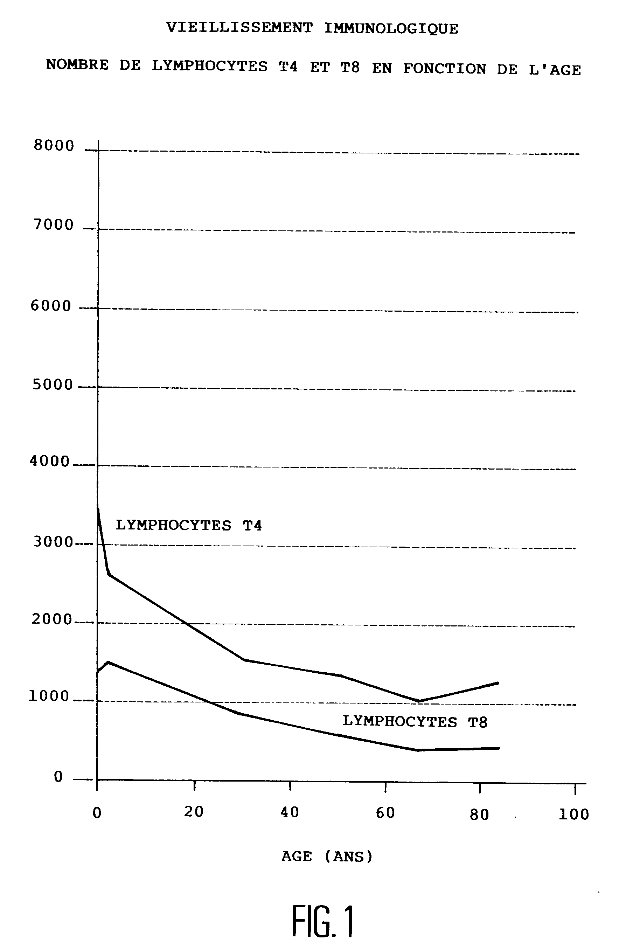

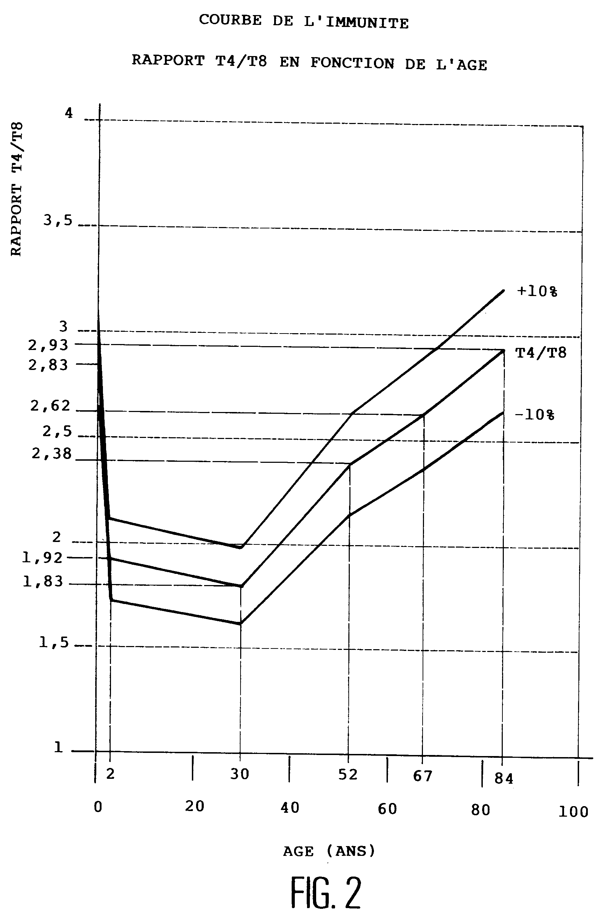

Les courbes du vieillissement immunologique font l'objet de la figure 1 annexée et la courbe de l'immunité fait l'objet de la figure 2 annexée.

Il apparaît ainsi que le rapport T4/T8 constitue un nouveau paramètre biologique dont le contrôle permet la surveillance de l'état du système immunitaire d'un sujet humain.

Aussi, la présente invention propose-t-elle un nouveau procédé de contrôle de l'immunité chez le sujet humain pour détecter une éventuelle déficience du système immunitaire, procédé qui se caractérisé en ce qu'il comprend les étapes consistant à :
(a) établir la courbe de l'évolution du rapport nombre de lymphocytes T4/nombre de lymphocytes T8 en fonction de l'âge sur des échantillons sanguins prélevés sur des sujets humains sains,
(b) déterminer, dans les mêmes conditions que (a), le rapport nombre de lymphocytes T4/nombre de lymphocytes T8 sur un échantillon sanguin prélevé sur un sujet humain en examen, et
(c) comparer le rapport nombre de lymphocytes T4/nombre de lymphocytes T8 obtenu à l'étape (b) à la courbe obtenue à l'étape (a), un rapport nombre de lymphocytes T4/nombre de lymphocytes T8 non en accord avec cette courbe étant révélateur d'une déficience du système immunitaire du sujet en examen.

Il est à noter que dans les étapes (a) et (b) ci-dessus, la détermination du nombre de lymphocytes T4 et T8 est de préférence effectuée au moyen de la technique de cytométrie en flux.

Le procédé ci-dessus défini est en outre caractérisé en ce qu'à l'étape (a), la courbe de l'évolution du rapport nombre de lymphocytes T4/nombre de lymphocytes T8 (T4/T8) en fonction de l'âge, est conforme à la figure 2 annexée.

Grâce au procédé selon l'invention, on dispose désormais d'un moyen sûr et simple de surveillance du système immunitaire et donc de dépistage d'éventuels syndromes immunodéficients chez tous sujets humains, y compris les sportifs de haut niveau, rendant possible une intervention médicale au stade le plus précoce de la maladie.

A titre illustratif, on décrira ci-après un exemple de détermination des populations des lymphocytes T4 et T8, par la méthode de cytométrie en flux

Sur un sujet sain de 37 ans, on prélève sur EDTA (agent anticoagulant) 100 µl de sang total. On ajoute 10 µl d'anticorps monoclonal pour le marquage de surface des cellules, commercialisé par la société américaine COULTER IMMUNOLOGY sous la marque Cyto-Stat/Coulter Clone T4-RD1/T8-FITC , puis on mélange la solution obtenue et l'incube pendant 10 minutes à la température ambiante.

On ajoute ensuite un réactif de lyse (par exemple 600 µl d'Immunoprep A - marque de fabrique de la société COULTER INC.) et homogénéise l'ensemble par agitation, ce réactif détruisant les hématies tout en laissant intacts les leucocytes. La lyse est ensuite interrompue par ajout, sous agitation, d'un réactif stabilisant (par exemple 250 µl d'Immunoprep B - marque de fabrique de la société COULTER INC.). Enfin, on ajoute éventuellement un réactif de fixation (par exemple 100 µl d'Immunoprep C - marque de fabrique de la Société COULTER INC.) qui permet de fixer les lymphocytes et offre donc la possibilité de reporter, si nécessaire, les mesures dans un délai de 24 à 48 heures (avec conservation de l'échantillon au réfrigérateur à 4° C).

L'échantillon est ensuite soumis à une lecture dans un cytomètre de flux EPICS PROFILE II de la société COULTER INC. [laser d'excitation d'une puissance de 15 mW à 488 nm ; débit de l'échantillon : 97 µl/mn ; pression de gaine : 52,7.10³ Pa (7,50 psi)]. Cette lecture donne les résultats suivants :
- nombre de lymphocytes T4 par mm³ de sang : 867
- nombre de lymphocytes T8 par mm³ de sang : 480

On en déduit un rapport T4/T8 de 1,81 avec une limite de précision de ± 10 %.

On donne également ci-après à titre d'exemples, les valeurs des rapports T4/T8 déterminées en suivant le mode opératoire ci-dessus, dans quelques cas de séropositivité et de mononucléose infectieuse :
- Mr. X. Olivier, âgé de 26 ans, séropositif : rapport T4/T8 = 0,5
- Mr. Y. Pascal, âgé de 35 ans, séropositif : rapport T4/T8 = 0,1
- Mr. Z. Jacques, âgé de 44 ans, séropositif : rapport T4/T8 = 0,1
- Mme. S. Hélène, âgée de 29 ans, atteinte de mononucléose infectieuse: rapport T4/T8 = 0,1,

tous les rapports T4/T8 étant déterminés avec une limite de précision d'environ ± 10 %.

## Revendications

1. Procédé de contrôle préventif de l'immunité chez le sujet humain pour détecter une éventuelle déficience du système immunitaire, caractérisé en ce qu'il comprend les étapes consistant à :
(a) établir la courbe de l'évolution du rapport nombre de lymphocytes T4/nombre de lymphocytes T8 en fonction de l'âge sur des échantillons sanguins prélevés sur des sujets humains sains,
(b) déterminer, dans les mêmes conditions que (a), le rapport nombre de lymphocytes T4/nombre de lymphocytes T8 sur un échantillon sanguin prélevé sur un sujet humain en examen, et
(c) comparer le rapport nombre de lymphocytes T4/nombre de lymphocytes T8 obtenu à l'étape (b) à la courbe obtenue à l'étape (a), un rapport nombre de lymphocytes T4/nombre de lymphocytes T8 non en accord avec cette courbe étant révélateur d'une déficience du système immunitaire du sujet en examen.

2. Procédé selon la revendication 1, caractérisé en ce que la détermination du nombre de lymphocytes T4 et du nombre de lymphocytes T8 aux étapes (a) et (b) est effectuée par cytométrie en flux.

3. Procédé selon la revendication 1 ou 2, caractérisé en ce que la courbe de l'évolution du rapport nombre de lymphocytes T4/nombre de lymphocytes T8 en fonction de l'âge est telle :
qu'à l'âge 0 (nouveaux-nés), ce rapport est de 2,83
qu'à l'âge de 2 ans, ce rapport est de 1,92,
qu'à l'âge de 30 ans, ce rapport est de 1,80,
qu'à l'âge de 52 ans, ce rapport est de 2,38,
qu'à l'âge de 67 ans, ce rapport est de 2,62, et
qu'à l'âge de 84 ans, ce rapport est de 2,93,
la limite de précision pour chacun de ces rapports étant de ± 10 %.

4. Procédé selon l'une quelconque des revendications précédentes, caractérisé en ce que la courbe de l'évolution du rapport nombre de lymphocytes T4/nombre de lymphocytes T8 en fonction de l'âge est conforme à la figure 2.

5. Utilisation du procédé selon l'une quelconque des revendications précédentes pour la surveillance du système immunitaire chez les sujets humains, y compris les sportifs de haut niveau.
